# EUROPÄISCHE PATENTSCHRIFT

(11) **EP 2 318 349 B1**
(45) Veröffentlichungstag und Bekanntmachung des Hinweises auf die Patenterteilung: **19.04.2017**
(21) Anmeldenummer: 09777498.8
(22) Anmeldetag: 29.07.2009
(51) Int. Cl.: C07C 45/50, C07C 47/02, C07C 45/82

(54) **VERFAHREN ZUR GEWINNUNG VON ALIPHATISCHEN C3- BIS C-10-ALDEHYDEN AUS HOCHSIEDENDEN STOFFEN MITELS THERMISCHER VERFAHREN**
METHOD FOR OBTAINING ALIPHATIC C3-TO C10- ALDEHYDES FROM HIGH-BOILING SUBSTANCES BY THERMAL PROCESSING
PROCÉDÉ D'OBTENTION D'ALDÉHYDES ALIPHATIQUES EN C3 À C10 À PARTIR DE COMPOSÉS À POINT D'ÉBULLITION ÉLEVÉ, PAR TRAITEMENT THERMIQUE

(30) Priorität: 28.08.2008 DE 102008044783
(43) Veröffentlichungstag der Anmeldung: 11.05.2011
(73) Patentinhaber: OXEA GmbH, 46147 Oberhausen (DE)
(72) Erfinder: BERGHAHN, Barbara, 4029 Düsseldorf (DE); GREB, Wolfgang, 46535 Dinslaken (DE); SCHÖPPER, Norbert, 47178 Duisburg (DE); ZGORZELSKI, Wolfgang, 46149 Oberhausen (DE)
(86) Internationale Anmeldenummer: PCT/EP2009/005470
(87) Internationale Veröffentlichungsnummer: WO 2010/022837

(56) Entgegenhaltungen:
- EP-A- 0 103 810

## Beschreibung

Die vorliegende Erfindung betrifft ein Verfahren zur Gewinnung von aliphatischen C₃-C₁₀-Aldehyden aus Hochsiedern, die bei der Hydroformylierung der entsprechenden C₂-C₉-Olefine in einem heterogenen Reaktionssystem unter Verwendung einer wässrigen Lösung anfallen, durch thermische Behandlung.

Als Hydroformylierung bezeichnet man die übergangsmetallkatalysierte Umsetzung von Olefinen mit Wasserstoff und Kohlenmonoxid zu Aldehyden und Alkoholen, die ein Kohlenstoff-Atom mehr als das eingesetzte Olefin enthalten. Die Hydroformylierung besitzt erhebliche wirtschaftliche und technische Bedeutung und Hydroformylierungsverfahren werden im großtechnischen Maßstab betrieben. Die dabei primär erhaltenen Aldehyde werden als solche verwendet oder stellen wertvolle Vorprodukte für die Gewinnung von beispielsweise Alkoholen, Carbonsäuren, Estern oder Aminen dar. Das Gemisch aus Wasserstoff und Kohlenmonoxid wird auch als Synthesegas bezeichnet.

Die Hydroformylierung wird durch Hydridometallcarbonyle, vorzugsweise solcher der Metalle der VIII. Nebengruppe des Periodensystems, katalysiert. Neben Kobalt, dem klassischen Katalysatormetall, werden seit einigen Jahren zunehmend Katalysatoren auf Basis von Rhodium eingesetzt. Im Gegensatz zu Kobalt gestattet es Rhodium, die Reaktion bei niedrigem Druck durchzuführen. Darüber hinaus werden bei Einsatz endständiger Olefine bevorzugt n-Aldehyde und nur in untergeordnetem Maße iso-Aldehyde bei der Reaktion zwischen Olefin, Kohlenmonoxid und Wasserstoff nach der Reaktionsgleichung 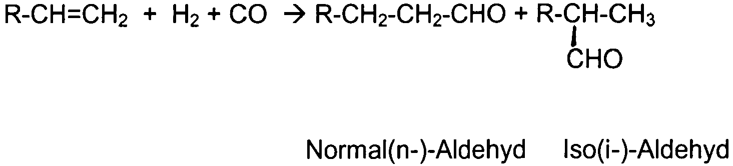 gebildet, wobei der organische Rest R sowohl geradkettig als auch verzweigt sein kann. Schließlich ist auch die Hydrierung der eingesetzten Olefine zu gesättigten Kohlenwasserstoffen in Gegenwart von Rhodium-Katalysatoren deutlich niedriger als bei Anwendung von Kobalt-Katalysatoren.

Industriell realisiert ist die Hydroformylierung olefinisch ungesättigter Verbindungen unter der katalytischen Wirkung von Rhodiumcarbonyl-Komplexen mit tertiären organischen Phosphinen oder Phosphiten als Liganden. Bei einer Prozessvariante arbeit man in homogener organischer Phase, d. h. eingesetztes Olefin, Katalysator und Reaktionsprodukte liegen gemeinsam in Lösung vor. Die Reaktionsprodukte werden aus dem Gemisch meist destillativ, seltener nach anderen Verfahren wie der Extraktion, abgetrennt. Das in homogener Phase durchgeführte Hydroformylierungsverfahren kann in Form eines Gaskreislaufverfahrens gemäß US 4,247,486 oder in Form eines Flüssigkeitskreislaufverfahrens gemäß US 4,148,830 ausgestaltet werden.

Eine weitere Verfahrensvariante ist durch die Gegenwart einer wässrigen Katalysatorphase gekennzeichnet, die Rhodiumcarbonyl-Komplexe und wasserlösliche organische Phosphine enthält. Diese Ausführungsform ist zum Beispiel aus DE-B-26 27 354 bekannt. Ihr besonderer Vorteil ist die leichte Trennung von organischem Reaktionsprodukt und wässriger Katalysatorphase, zum Beispiel durch Phasentrennung. Diese Trennung erfolgt schonend und ohne Anwendung thermischer Verfahrensschritte und Verluste lassen sich vermeiden, die durch Folgereaktionen der entstandenen Aldehyde eintreten können. Weiterhin erzielt man sehr hohe Ausbeuten und bei der Verwendung unverzweigter endständiger Olefine erhält man ganz überwiegend unverzweigte Aldehyde. Aufgrund des Vorliegens einer flüssigen organischen Phase und einer wässrigen Katalysatorphase bezeichnet man diese Hydroformylierungsvariante auch als heterogenes oder zweiphasiges Verfahren. Eine bewährte Ausführungsform dieser Arbeitsweise ist beispielweise in der EP-B1-0 103 810 beschrieben.

Sowohl das in homogener Phase durchgeführte Hydroformylierungsverfahren als auch das heterogene Hydroformylierungsverfahren, das auch als Ruhrchemie/Rhone-Poulenc-Verfahren bekannt ist, haben sich in der großtechnischen Anwendung etabliert und werden in der Literatur, beispielsweise von C.D.Frohning, C.W.Kohlpaintner in B. Cornils/W.A.Herrmann, Applied Homogeneous Catalysis with Organometallic Compounds, Volume 1, 1. Auflage, Seiten 29-104, VCH, Weinheim, 1996, ausführlich behandelt.

Es ist bekannt, dass die in der Hydroformylierungsreaktion als Hauptprodukt gebildeten n-Aldehyde und die in geringeren Mengen gebildeten iso-Aldehyde schon während des Hydroformylierungsprozesses Aldol-Kondensationsprodukte bilden. US 4,247,486 beschreibt beispielhaft die Bildung von Dimeren und Trimeren aus der Selbstkondensation von n-Butyraldehyd sowie aus der Mischkondensation von n- und iso-Butyraldehyd. Ferner können die Aldol-Kondensationsprodukte einer Disproportionierungsreaktion gemäß der Tischtschenkoreaktion unterliegen. Im homogen durchgeführten Hydroformylierungsprozess eignen sich diese Kondensationsprodukte als Lösungsmittel für den Rhodium-Katalysator und ihre Gegenwart wirkt sich auch vorteilhaft auf die Stabilität des gelösten Rhodium-Katalysators aus. Diese Kondensationsprodukte können in einer Menge von mehr als 70 Gew.-%, bezogen auf die gesamte organische Lösung, zugegen sein. Im stationären Zustand wird der Reaktor so betrieben, dass die über den Gaskreislauf abgeführte Menge an Kondensationsprodukten der während der Hydroformylierungsreaktion gebildeten Menge entspricht und sich so eine stabile Massenbilanz einstellt.

Auch bei der Hydroformylierung von Olefinen nach dem Zweiphasen-Verfahren bilden sich aus den n- und iso-Aldehyden Aldol-Kondensationsprodukte und hochsiedende Verbindungen, beispielsweise Tischtschenkoester. Nach der Lehre von US 4,684,750 A1 wird zunächst Propylen nach dem Zweiphasen-Verfahren hydroformyliert. Die nach Phasentrennung erhaltene organische Phase wird anschließend destilliert, wobei gelöste Gase wie Kohlenmonoxid, Wasserstoff, Propan und Propylen sowie auch die Hauptmenge an iso-Butyraldehyd über den Kopf der Destillationskolonne abgetrennt werden. Der Destillationsrückstand enthält überwiegend n-Butyraldehyd und geringe Mengen an iso-Butyraldehyd, n- und iso-Butanol, Aldolen und hochsiedenden Komponenten. Da nach dem Zweiphasen-Verfahren die wässrige Katalysator haltige Phase zunächst abgetrennt wird, ist der nach Abtrennung gelöster Gase anfallende Destillationsrückstand frei von dem Hydroformylierungskatalysator. Hingegen enthalten die bei der homogenen Reaktionsführung nach Aldehydabtrennung anfallenden Hochsieder noch den Hydroformylierungskatalysator.

Während nach der homogenen Verfahrensführung die Hochsiederbildung bis zu einem stationären Zustand gewünscht ist, um ihre Lösungsmitteleigenschaften und ihre stabilisierende Wirkung auf den Hydroformylierungskatalysator zu nutzen, bedeutet die Hochsiederbildung bei dem Zweiphasen-Verfahren einen Nachteil, weil in den Hochsiedern Äquivalente der gewünschten Aldehyde gebunden sind und durch ihre Bildung eine erhebliche Menge an Wertprodukt verloren geht. Ebenfalls ist in den Hochsiedern noch ein erheblicher Anteil an den gewünschten Aldehyden physikalisch gelöst. Zudem ist die Entsorgung der Hochsieder aufwendig und teuer. Es besteht daher der Bedarf an einem Verfahren, aus den Hochsiedern, die bei der Hydroformylierung von Olefinen nach dem Zweiphasen-Verfahren anfallen, den entsprechenden n- und iso-Aldehyd zurückzugewinnen.

Die Erfindung besteht daher in einem Verfahren zur Gewinnung von aliphatischen C₃- bis C₁₀-Aldehyden aus Hochsiedern, die bei der Hydroformylierung der entsprechenden C₂- bis C₉-Olefine in einem heterogenen Reaktionssystem unter Verwendung einer wässrigen Lösung anfallen, die Übergangsmetallkomplexverbindungen der Gruppe VIII des Periodensystems der Elemente mit wasserlöslichen, organischen Phosphor(III)verbindungen gelöst enthält, und wobei man das dem Hydroformylierungsreaktor entnommene Reaktionsgemisch in eine wässrige, Übergangsmetallkomplexverbindung enthaltende Phase und in eine organische Phase trennt, die abgetrennte organische Phase nach Entfernung flüchtiger Bestandteile in einer ersten Destillationskolonne destilliert und ein Kopfprodukt und ein Sumpfprodukt gewinnt. Es ist dadurch gekennzeichnet, dass man das so gewonnene Sumpfprodukt in eine zweite Destillationskolonne gibt und als Kopfprodukt den n-Aldehyd gewinnt und als Sumpfprodukt ein Gemisch aus dem n-Aldehyd und den Hochsiedern abführt und dass man dieses Sumpfprodukt auf eine dritte Destillationskolonne gibt, die unter solchen Bedingungen betrieben wird, dass noch keine Spaltung der Hochsieder in n-und iso-Aldehyd einsetzt, und dass man das Sumpfprodukt der dritten Destillationskolonne auf eine vierte Destillationskolonne gibt, in der das zugeführte Sumpfprodukt aus der dritten Destillationskolonne bei erhöhter Temperatur gespalten wird, und ein Gemisch aus n- und iso-Aldehyd als Kopfprodukt abzieht.

Die im erfindungsgemäßen Verfahren eingesetzten Hochsieder fallen bei der Hydroformylierungsreaktion der entsprechenden C₂- bis C₉-Olefine zu aliphatischen C₃- bis C₁₀-Aldehyden nach dem heterogenen oder Zweiphasen-Verfahren an. Ihre Bildung erfolgt zum einen im Reaktor während der Hydroformylierungsreaktion. Hochsieder entstehen aber auch in nachfolgenden destillativen Aufarbeitungsschritten zu den gereinigten n- und iso-Aldehyden. Da bei der heterogenen Reaktionsführung der Hydroformylierungskatalysator in der wässrigen Phase verbleibt, die sich aus dem Reaktionsgemisch nach Verlassen des Hydroformylierungsreaktors und Entfernung flüchtiger Bestandteile abscheidet, sind in den im Zweiphasen-Verfahren anfallenden Hochsiedern keine Übergangsmetallkomplexverbindungen mehr enthalten, im Gegensatz zu Hochsiederströmen, die in der homogenen Verfahrensführung bei der destillativen Abtrennung der gewünschten Aldehyde als Rückstände anfallen. Unter dem Begriff Hochsieder werden Aldol-Kondensationsprodukte aus der Selbstkondensation der n-Aldehyde sowie aus der gemischten Kondensation des n- und iso-Aldehyds verstanden, sowie daraus nach der Tischtschenko-Reaktion gebildete Esterverbindungen und weitere Sauerstoff haltige hochsiedende Verbindungen.

Bei der heterogen durchgeführten Hydroformylierungsreaktion wird zunächst der Reaktoraustrag in einen Phasentrenner gegeben, in dem die Gasphase, im Wesentlichen Synthesegas, Olefin, aus dem Olefin gebildeter gesättigter Kohlenwasserstoff, von den Flüssigprodukten getrennt und über einen Kompressorkreislauf wieder dem Reaktor zugeführt wird. Die im Phasentrenner abgeschiedene Flüssigkeit wird hier außerdem in die wässrige Katalysatorphase und die organische Produktphase getrennt. Das organische Rohprodukt wird auf eine Strippkolonne gegeben und mit im Gegenstrom herangeführten Synthesegas behandelt. Das mit dem im Rohprodukt gelösten Olefin beladene Synthesegas wird in den Hydroformylierungsreaktor zurückgeführt, während das rohe, organische Produkt aus der Strippkolonne anschließend in eine erste Destillationskolonne gegeben wird.

In dieser ersten Destillationskolonne wird das olefinfreie, rohe, organische Produkt in den n- und iso-Aldehyd getrennt. Aufgrund der vergleichsweise geringen Siedepunktdifferenzen zwischen n- und iso-Aldehyd hat die erste Destillationskolonne eine hohe Trennleistung aufzuweisen und sie besitzt dementsprechend eine hohe Zahl an theoretischen Böden. Iso-Aldehyd wird als flüchtige Komponente am Kopf abgezogen. Der angefallene Destillationsrückstand enthält überwiegend den n-Aldehyd, geringe Mengen an iso-Aldehyd und an durch Hydrierung der Aldehyde gebildeten Alkoholen sowie die gebildeten Hochsieder. Der Hochsiederanteil im Destillationssumpf liegt im Allgemeinen in einem Bereich von 0,5 bis 1,5 Gew.-%, bezogen auf den gesamten Destillationsrückstand. In einer weiteren Ausgestaltung des erfindungsgemäßen Verfahrens kann man im ersten Destillationsschritt zur Abtrennung des iso-Aldehyds anstelle einer ersten Destillationskolonne auch eine Mehrzahl von hintereinander geschalteten Destillationskolonnen verwenden. Bei dieser Kolonnenschaltung wird das Kopfprodukt aus einer Destillationskolonne auf eine folgende Destillationskolonne gegeben, bei der das hier anfallende Kopfprodukt wiederum auf eine weitere Destillationskolonne gegeben wird. Schließlich wird das Kopfprodukt der letzten Destillationskolonne, das überwiegend aus iso-Aldehyd besteht, nach an sich bekannten Verfahren weiterverarbeitet. Die bei den jeweiligen Kolonnen anfallenden Sumpfprodukte werden vereinigt. Der Hochsiederanteil im vereinigten Destillationssumpf liegt im Allgemeinen in einem Bereich von 0,5 bis 2,5 Gew.-%.

Das Sumpfprodukt aus der ersten Destillationskolonne oder die vereinigten Sumpfprodukte aus dem ersten Destillationsschritt werden auf eine zweite Destillationskolonne gegeben, die man auch als Reinaldehyddestillationskolonne bezeichnet. Die Betriebsbedingungen dieser Kolonne entsprechen dabei den Bedingungen der ersten Destillationskolonne. Über Kopf wird gereinigter n-Aldehyd abgezogen, während über den Sumpf ein Gemisch aus n-Aldehyd und Hochsiedern ausgeschleust wird. Da diese Destillationskolonne nur zur Hochsiederabtrennung dient, kann ein vergleichsweise niedriges Rücklaufverhältnis von 1:2 bis 1:10, vorzugsweise von 1:3 bis 1:6 eingestellt werden. Das am Sumpf der zweiten Destillationskolonne abgezogene Gemisch aus n-Aldehyd und Hochsiedern wird anschließend auf eine dritte Destillationskolonne geführt.

Die Bedingungen, unter denen diese dritte Destillationskolonne betrieben wird, sind innerhalb enger Grenzen festzulegen. Die thermische Behandlung erfolgt in der Weise, dass es noch nicht zu einer Spaltung der Hochsieder kommt, sondern dass die Restmengen des physikalisch gelösten n-Aldehyds verdampfen und über den Kopf abgezogen werden können. Wesentlich ist, dass an dieser Stelle des Aufarbeitungsverfahrens nur n-Aldehyd als Kopfprodukt abgezogen wird und die Hochsiederspaltung, die auch zu einer gewissen Bildung an iso-Aldehyd führt, noch nicht eintritt.

Im Allgemeinen hat die dritte Destillationskolonne von 30 bis 50 theoretische Böden. Der über Kopf abgeführte n-Aldehyd kann anschließend mit dem am Kopf der zweiten Destillationskolonne angefallenen n-Aldehyd vereinigt und weiterverarbeitet werden.

In einer bevorzugten Ausgestaltung des erfindungsgemäßen Verfahrens werden in diese dritte Destillationskolonne gleichzeitig auch entsprechende Gemische aus n-Aldehyd und Hochsiedern eingespeist, die aus parallel betriebenen und nach dem heterogenen Hydroformylierungsverfahren arbeitenden Reaktoren abgeführt werden.

Das Sumpfprodukt der dritten Destillationskolonne wird anschließend auf eine vierte Destillationskolonne gegeben. Im Allgemeinen hat die vierte Destillationskolonne von 20 bis 40 theoretische Böden. Die Bedingungen werden so eingestellt, dass es zu einer thermischen Spaltung der Hochsieder kommt. Die Hochsiederspaltung erfolgt auf thermischem Wege und ohne Zugabe von sauren oder alkalischen Reagenzien, die beispielsweise gemäß der DE 102 52 173 B3 zur Spaltung der bei der Herstellung von 2-Ethylhexanol anfallenden Hochsiedern eingesetzt werden. Als Kopfprodukt wird ein Gemisch aus den entsprechenden n- und iso-Aldehyden, das noch geringe Mengen an den entsprechenden Alkoholen enthält, abgezogen und der anfallende hochsiedende Destillationsrückstand ausgeschleust.

Der als Sumpf aus der vierten Destillationskolonne abgeführte hochsiedende Rückstand wird aus dem Prozess entfernt und anderweitig verwertet, beispielsweise als Lösungsmittel oder durch Verbrennung zur Energiegewinnung genutzt. Das über Kopf der vierten Destillationskolonne abgezogene Gemisch aus n- und iso-Aldehyd kann beispielsweise in die erste Destillationskolonne zurückgeführt werden und in den entsprechenden iso-Aldehyd und n-Aldehyd aufgetrennt werden.

Bei der ersten, zweiten, dritten und vierten Destillationskolonne handelt es sich um übliche Destillationskolonnen aus gängigen Materialien, die auch Einbauten, wie Böden oder Packungen enthalten können. Vorzugsweise werden die Destillationskolonnen kontinuierlich betrieben.

Die Temperatur- und Druckbedingungen, unter denen die Destillationskolonnen betrieben werden, hängen von den Stoffdaten der verwendeten Olefine und der daraus erhaltenen n- und iso-Aldehyde ab. Setzt man Propylen als Olefin nach dem heterogenen Hydroformylierungsverfahren um, so wird die erste Destillationskolonne im Allgemeinen bei einer Kopftemperatur von 60 bis 70°C, bei einer Sumpftemperatur von 90 bis 100°C und bei Normaldruck betrieben.

Ihre Arbeitsweise wird schematisch in Multiphase Homogeneous Catalysis, Volume 1, Wiley-VCH Verlag GmbH & Co. KGaA, 1. Auflage, Weinheim 2005, Kapitel "C3 Process Description", Seiten 167-169 dargestellt und im Detail in US 4,684,750 A1 beschrieben. Die erste Destillationskolonne weist 90 bis 110 theoretische Böden auf. Entsprechende Bedingungen im ersten Destillationsschritt werden ebenfalls eingestellt, wenn man anstelle der ersten Destillationskolonne mehrere hintereinandergeschaltete Destillationskolonnen verwendet. Die Betriebsbedingungen der zweiten Destillationskolonne entsprechen den Bedingungen der ersten Destillationskolonne. Es ist jedoch empfehlenswert, gegenüber den Destillationsbedingungen in der ersten Destillationskolonne eine um bis zu 10°C, vorzugsweise um bis zu 5°C niedrigere Kopftemperatur einzustellen. Die dritte Destillationskolonne wird bei einer Kopftemperatur von 75 bis 95°C, vorzugsweise von 80 bis 90°C, bei einer Sumpftemperatur von 105 bis 120°C, vorzugsweise 110 bis 115°C, und bei Normaldruck betrieben. Die am Kopf der zweiten und dritten Destillationskolonne anfallenden, gereinigten n-Butyraldehydströme können vereinigt werden und beispielsweise zu n-Butanol hydriert, zu n-Buttersäure oxidiert oder unter Alkalieinfluss zu 2-Ethylhexenal kondensiert werden, das anschließend zu 2-Ethylhexanol hydriert wird. Die vierte Destillationskolonne wird bei einer Kopftemperatur von 70 bis 100°C, vorzugsweise von 80 bis 90°C, bei einer Sumpftemperatur von 130 bis 160°C, vorzugsweise von 140 bis 150°C, und bei Normaldruck betrieben, und über Kopf wird ein Gemisch aus n- und iso-Butyraldehyd abgezogen.

Wird ein anderes Olefin als Propylen nach dem Zweiphasenverfahren eingesetzt, sind die Betriebsbedingungen der Destillationskolonnen entsprechend den Stoffdaten der eingesetzten Ausgangsolefine und der erhaltenen Aldehyde anzupassen. Die jeweils zugeschnittenen Betriebsbedingungen lassen sich durch Routineversuche ermitteln.

Die erfindungsgemäß zu behandelnden Hochsieder fallen bei der Hydroformylierung von C₂- bis C₉-Olefinen nach dem Zweiphasen-Verfahren oder nach dem heterogenen Verfahren an, eine Umsetzung, die zum Beispiel in der DE-B-26 27 354 beschrieben ist. Dieser Prozess ist charakterisiert durch das Vorliegen einer organischen Phase, die das olefinische Ausgangsmaterial und das Reaktionsprodukt enthält, und einer wässrigen Phase, in der der Katalysator gelöst ist. Als Katalysatoren werden wasserlösliche Komplexverbindungen von Übergangsmetallen der Gruppe VIII des Periodensystems des Elements eingesetzt, die wasserlösliche organischen Phosphor(III)-Verbindungen als Liganden enthalten. Beispiele für wasserlösliche Phosphor(III)-Verbindungen, die mit den Übergangsmetallen Komplexverbindungen bilden, sind Triarylphosphine, Trialkylphosphine, gemischte aliphatische-aromatische Phosphine und arylierte bzw. alkylierte Diphosphine, deren organische Reste Sulfonsäuregruppen oder Carboxylgruppen enthalten. Ihre Herstellung und Anwendung ist zum Beispiel aus DE-B 26 27 354, EP-B1-0 103 810, EP-B1-0 163 234 und EP-A1-0 571 819 bekannt. Weitere Gruppen geeigneter Verbindungen sind sulfonierte oder carboxylierte organische Phosphite sowie heterocyclische Verbindungen des dreiwertigen Phosphors, die zum Beispiel aus EP-A1-0 575 785 und EP-A1-0 646 588 bekannt sind.

Als sulfonierte Arylphosphine eignen sich besonders sulfonierte Triarylphosphine der allgemeinen Formel (I) 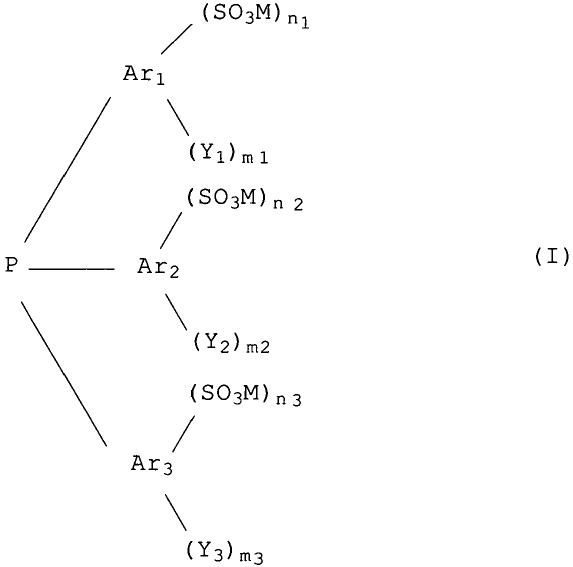 in der Ar¹, Ar² und Ar³ gleiche oder verschiedene Arylgruppen mit 6 bis 14 Kohlenstoffatomen bedeuten, die Substituenten Y₁, Y₂ und Y₃ gleiche oder verschiedene geradkettige oder verzweigte Alkyl- oder Alkoxyreste mit 1 bis 4 Kohlenstoffatomen, Chlor, Brom, die Hydroxyl-, Cyanid- oder Nitrogruppe bedeuten, ferner für die Aminogruppe der Formel NR¹R² stehen, in der die Substituenten R¹ und R² gleich oder verschieden sind und Wasserstoff, geradkettige oder verzweigte Alkylgruppen mit 1 bis 4 Kohlenstoffatomen bedeuten, in der M für Lithium, Natrium, Kalium, Magnesium, Calcium oder Barium steht, in der m₁, m₂ und m₃ gleich oder verschieden sind und ganze Zahlen von 0 bis 5 bedeuten, in der n₁, n₂ und n₃ gleich oder verschieden sind und für ganze Zahlen von 0 bis 3 stehen, wobei mindestens eine der Zahlen n₁, n₂ und n₃ gleich oder größer 1 ist.

Zu den Triarylphosphinen zählen bevorzugt solche Triarylphosphine, bei denen die Gruppen Ar¹, Ar², Ar³ Phenylgruppen sind; Y₁, Y₂ und Y₃ für die Methyl-, die Ethylgruppe, für die Methoxy-, Ethoxygruppe und/oder für ein Chloratom stehen; und die kationischen Reste M anorganische Kationen von Natrium, Kalium, Calcium und Barium sind. Insbesondere geeignet sind solche Triarylphosphine, bei denen Ar¹, Ar², Ar³ jeweils eine Phenylgruppe bedeuten, m₁, m₂, m₃ gleich 0 sind, n₁, n₂ und n₃ gleich 0 oder 1 sind und n₁+n₂+n₃ zusammen 1 bis 3 ausmachen, und bei denen die Sulfonat-Gruppen in meta-Stellung stehen.

Ein für die Durchführung des heterogenen Hydroformylierungsverfahrens geeignetes Gemisch an (Sulfophenyl)-diphenylphosphin, Di-(sulfophenyl)phenylphosphin und Tri(sulfophenylphosphin) fällt bei der Sulfonierung von Triphenylphosphin an, wie zum Beispiel aus DE-OS 26 27 354 bekannt. Im Stand der Technik wird (Sulfophenyl)diphenylphosphin als TPPMS, Di-(sulfophenyl)phenylphosphin als TPPDS und Tri(sulfophenyl)phosphin als TPPTS abgekürzt.

Besonders vorteilhaft ist der Einsatz wasserlöslicher Komplexverbindungen des Rhodiums, obwohl die Verwendung anderer katalytisch aktiver Übergangsmetallverbindungen der Gruppe VIII des Periodensystems der Elemente nicht ausgeschlossen wird. So kann man auch wasserlösliche Komplexverbindungen von Kobalt, Iridium, Nickel, Palladium, Platin, Eisen oder Ruthenium verwenden, und besonders wasserlösliche Komplexverbindungen des Kobalts, Iridiums und Platins haben sich als wirksame Hydroformylierungskatalysatoren erwiesen.

Die Bedingungen, unter denen die Hydroformylierung abläuft, können innerhalb weiter Grenzen variieren und den individuellen Gegebenheiten angepasst werden. Sie hängen u.a. vom Einsatzmaterial, vom ausgewählten Katalysatorsystem und vom angestrebten Umsetzungsgrad ab. Üblicherweise führt man die Hydroformylierung der Einsatzstoffe bei Temperaturen von 70 bis 150°C durch. Bevorzugt hält man Temperaturen von 100 bis 150°C und insbesondere von 110 bis 140°C ein. Der Gesamtdruck erstreckt sich über einen Bereich von 0,5 bis 10 MPa, vorzugsweise 1 bis 6 MPa und insbesondere 1,5 bis 5 MPa. Das molare Verhältnis von Wasserstoff zu Kohlenmonoxid bewegt sich üblicherweise zwischen 1:10 und 10:1, Mischungen, die Wasserstoff und Kohlenmonoxid im molaren Verhältnis 3:1 bis 1:3, insbesondere etwa 1:1, enthalten, sind besonders geeignet.

Die Übergangsmetall-Konzentration beträgt 20 bis 1000 Gew.-ppm, vorzugsweise 50 bis 800 Gew.-ppm und insbesondere 100 bis 600 Gew.-ppm, jeweils bezogen auf die wässrige Katalysatorlösung. Obgleich es möglich ist, als Katalysator die stöchiometrisch zusammengesetzte Übergangsmetall-Phosphor-Komplexverbindung einzusetzen, arbeitet man üblicherweise in Gegenwart von überschüssigem Phosphorliganden, d.h. Ligand, der mit dem Übergangsmetall keine komplexe Bindung eingegangen ist. Je mol Übergangsmetall wendet man bevorzugt 10 bis 300 mol Phosphor in Form einer wasserlöslichen organischen Phosphorverbindung an. Besonders bewährt haben sich molare Verhältnisse von Übergangsmetall zu Phosphor im Bereich von 1:50 bis 1:150. Der Übergangsmetall-Phosphor-Komplexkatalysator braucht nicht einheitlich zusammengesetzt sein, sondern kann z.B. aus einem Gemisch von Übergangsmetall-Komplexverbindungen bestehen, die sich durch die Art der Phosphor-Liganden unterscheiden. Ebenso kann der in der wässrigen Katalysatorlösung enthaltene freie Phosphor-Ligand aus einem Gemisch unterschiedlicher wasserlöslicher organischer Phosphorverbindungen zusammengesetzt sein. Vorzugsweise verwendet man Rhodium als Übergangsmetall.

Wird ein anderes Übergangsmetall der Gruppe VIII des Periodensystems der Elemente als Rhodium als katalytisch aktives Metall eingesetzt, so bewegt sich die Konzentration an Übergangsmetall sowie das molare Verhältnis von Übergangsmetall zu Phosphor in den Bereichen, die man auch bei Rhodium wählt. Die jeweiligen optimalen Werte lassen sich in Abhängigkeit von dem jeweils eingesetzten Übergangsmetall durch einfache Routineversuche ermitteln.

Man bildet den Katalysator üblicherweise aus den Komponenten Übergangsmetall oder Übergangsmetallverbindung, organische Phosphorverbindung und Synthesegas unter den Bedingungen der Hydroformylierungsreaktion im Reaktionsgemisch. Es ist aber auch möglich, den Katalysator zunächst zu präformieren und ihn anschließend der eigentlichen Hydroformylierungsstufe zuzuführen. Die Bedingungen der Präformierung entsprechen dabei im Allgemeinen den Hydroformylierungsbedingungen.

Als C₂- bis C₉-Olefine setzt man aliphatische, geradkettige oder verzweigte Olefine mit einer end- oder innenständigen Doppelbindung ein, vorzugsweise Ethylen, Propylen, Buten-1 oder Gemische enthaltend Buten-1 und Buten-2. Bei höheren Olefinen, die in der wässrigen Katalysatorlösung nur sehr wenig löslich sind, kann es sich empfehlen, der wässrigen Katalysatorlösung ein Phasentransferreagenz oder Lösungsvermittler zuzusetzen, um den Umsatz je Zeiteinheit zu erhöhen. Lösungsvermittler verändern die physikalischen Eigenschaften der Grenzflächen zwischen den beiden flüssigen Phasen und erleichtert den Übergang des organischen Reaktanten in die wässrige Katalysatorlösung.

Als Lösungsvermittler sind Verbindungen bekannt, deren hydrophile Gruppen ionisch (anionisch oder kationisch) oder nicht ionisch sind. Zu den anionenaktiven Verbindungen gehören Natrium-, Kalium- oder Ammoniumsalze von Carbonsäuren, vorzugsweise solcher mit 8 bis 20 Kohlenstoffatomen und insbesondere von gesättigten Fettsäuren mit 12 bis 18 Kohlenstoffatomen, ferner Alkylsulfate, Alkylbenzolsulfonate und Alkylbenzolphosphate. Beispiele für kationische Lösungsvermittler sind Tetraalkylammonium- und N-Alkylpyridiniumsalze. Die nichtionischen Phasentransferreagenzien dissoziieren in wässriger Lösung nicht in Ionen. Zu ihnen zählen Alkylpolyethylenglykole, Alkylphenylpolyethylenglykole, Fettsäurealkylolamine und Trialkylaminoxide. Ferner finden Ampholyte wie Aminocarbonsäuren, Betaine und Sulfobetain als Lösungsvermittler Anwendung. Entsprechende Verfahren sind z.B. aus EP-B1-0 157 316 bekannt.

Es können auch Übergangsmetallkomplexverbindungen eingesetzt werden, die gleichzeitig Katalysator und Phasentransferreagenz sind. Eine solche Arbeitsweise ist zum Beispiel Gegenstand der EP-B1-0 163 234.

Der Einsatz von Lösungsvermittlern zur Zweiphasen-Hydroformylierung von niederen Olefinen, beispielsweise von Propylen oder von Buten-1 und Buten-2 enthaltenden Gemischen ist ebenfalls möglich und aus WO 98/30527 A1 bekannt.

Zur Herstellung des Hydroformylierungskatalysators gelangt das Übergangsmetall der Gruppe VIII des Periodensystems der Elemente, insbesondere Rhodium, entweder in metallischer Form oder als Verbindung zum Einsatz. In metallischer Form verwendet man das Übergangsmetall entweder als feinverteilte Partikel oder in dünner Schicht auf einem Träger, wie Aktivkohle, Calciumcarbonat, Aluminiumsilikat, Tonerde niedergeschlagen. Als Übergangsmetallverbindungen eignen sich Salze aliphatischer Mono- und Polycarbonsäuren, wie Übergangsmetall-2-Ethylhexanoate, -Acetate, -Oxalate, - Propionate oder -Malonate. Weiterhin können Salze anorganischer Wasserstoff- und Sauerstoffsäuren, wie Nitrate oder Sulfate, die verschiedenen Übergangsmetalloxide oder auch Übergangsmetallcarbonylverbindungen, wie Rh₃(CO)₁₂, Rh₆(CO)₁₆, CO₂(CO)₈, CO₄(CO)₁₆, Fe(CO)₅, Fe₂(CO)₉, Ir₂(CO)₈, Ir₄(CO)₁₂ oder Übergangsmetall-Komplexverbindungen, z.B. Cyclopentadienylrhodiumverbindungen, Rhodiumacetylacetonat, Cyclopentadienylkobalt-Cyclooctadien-1,5, Fe(CO)₃-Cyclooctadien-1,5, [RhCl(Cyclooctadien-1,5]₂ oder PtCl₂(Cyclooctadien-1,5) eingesetzt werden.

Übergangsmetallhalogenverbindungen kommen wegen ihres korrosiven Verhaltens der Halogenidionen weniger in Betracht.

Bevorzugt werden Übergangsmetalloxide und insbesondere Übergangsmetallacetate und -2-ethylhexanoate. Als besonders geeignet hat sich Rhodiumoxid, Rhodiumacetat, Rhodium-2-ethylhexanoat, Kobaltoxid, Kobaltacetat und Kobalt-2-ethylhexanoat erwiesen.

Die Hydroformylierungsstufe kann sowohl absatzweise als auch kontinuierlich durchgeführt werden. Hinsichtlich ihrer verfahrenstechnischen und apparativen Ausgestaltung kann man sich innerhalb weiter Grenzen bewegen. Eine bewährte Ausführungsform der heterogenen Hydroformylierung unter Verwendung einer wässrigen Katalysatorphase ist in der EP-B1-0 103 810 beschrieben. Der Reaktionsaustrag der Hydroformylierungsstufe wird in einem Phasentrenner in die organische Produktphase und in die wässrige Katalysatorlösung aufgetrennt. Es hat sich als zweckmäßig erwiesen, die Katalysatorlösung im Kreis zu führen.

Die erhaltene rohe organische Produktphase wird zunächst nach bekannten Verfahren in einer Stripp-Kolonne von gelösten Olefinen befreit und anschließend nach dem erfindungsgemäßen Verfahren behandelt. Aus den bei der heterogenen Hydroformylierungsstufe gebildeten Hochsiedern lassen sich die entsprechenden n- und iso-Aldehyde zurückgewinnen.

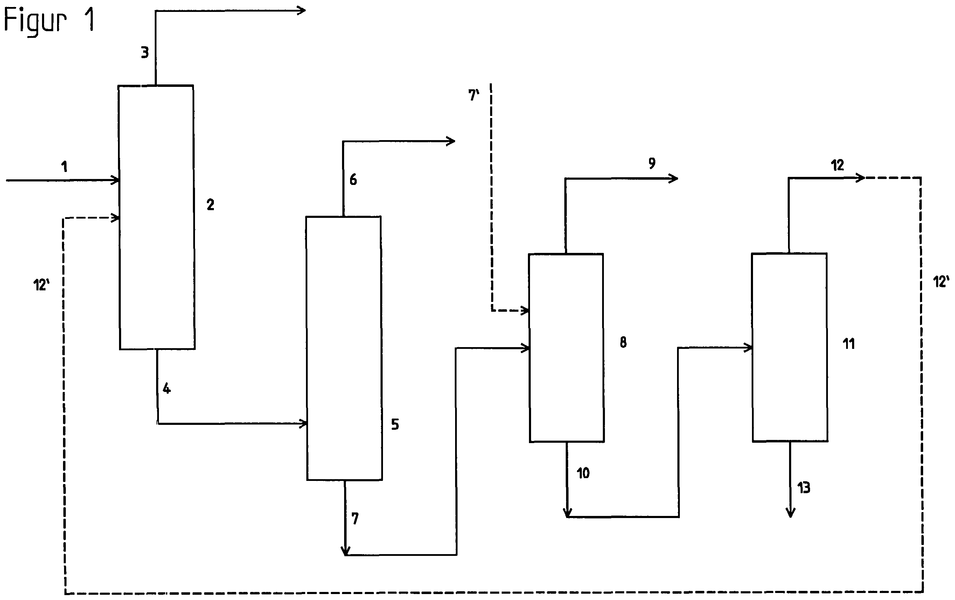

Figur 1 zeigt ein Prinzipschema für die erfindungsgemäße Behandlung der Hochsieder. Das über die Leitung (1) herangeführte organische, rohe, aber bereits gestrippte Hydroformylierungsprodukt wird auf eine erste Destillationskolonne (2) gegeben, bei der am Kopf über Leitung (3) ein Stoffstrom abgezogen wird, der die überwiegende Menge an iso-Aldehyd enthält. Der über Leitung (3) abgezogene iso-Aldehyd wird nach an sich bekannten Verfahren weiter aufgearbeitet. Anstelle der ersten Destillationskolonne (2) können auch mehrere, hintereinander geschaltete Destillationskolonnen im ersten Destillationsschritt betrieben werden, um n- und iso-Aldehyd aufzutrennen. Über den Sumpf der ersten Destillationskolonne (2) wird mit der Leitung (4) ein Stoffstrom abgeführt, in dem neben dem n-Aldehyd auch noch die Hochsieder vorliegen. Der Betrieb dieser Destillationskolonne ist beispielsweise aus US 4,684,750 A1 bekannt. Der Sumpfabzug der ersten Destillationskolonne wird auf eine zweite Destillationskolonne (5) gegeben, bei der am Kolonnenkopf über Leitung (6) gereinigter n-Aldehyd abgezogen wird, während über den Sumpf der Kolonne (5) ein Gemisch aus überwiegend n-Aldehyd enthaltend die gebildeten Hochsieder mittels Leitung (7) ausgeschleust und auf die dritte Destillationskolonne (8) gegeben wird, die unter solchen Bedingungen betrieben wird, dass die Hochsiederspaltung noch nicht eintritt und physikalisch gelöster n-Aldehyd am Kolonnenkopf über die Leitung (9) abgeführt wird. Die über Leitung (10) am Sumpf der dritten Destillationskolonne (8) abgeführten Hochsieder werden auf eine vierte Destillationskolonne (11) gegeben, in der die Hochsieder thermisch gespalten werden. Über Leitung (12) werden die flüchtigen Spaltprodukte, im Wesentlichen n- und iso-Aldehyd und geringe Mengen an den entsprechenden Alkoholen, abgezogen und die zurückbleibenden Hochsieder über die Leitung (13) ausgeschleust. Der über die Leitungen (6) und (9) abgezogene gereinigte n-Aldehyd wird nach an sich bekannten Verfahren weiterverarbeitet.

In einer weiteren Ausgestaltung des erfindungsgemäßen Verfahrens kann das über Leitung (12) abgezogene Gemisch enthaltend n- und iso-Aldehyd wieder in die Destillationskolonne (2) zurückgeführt werden, eine Verfahrensvariante, die in dem Prinzipschema nach Figur 1 mit der Leitung (12') angedeutet wird. Ebenfalls können der dritten Destillationskolonne (8) über die Leitung (7') entsprechende Gemische aus n-Aldehyd und Hochsiedern zugeführt werden, die aus weiteren, parallel betriebenen Hydroformylierungsreaktoren analog ausgeschleust werden.

Durch das erfindungsgemäße Verfahren kann der infolge der Bildung von Aldol-Kondensationsprodukten und Hochsiedern eintretende Aldehydverlust reduziert werden und, bezogen auf 100 Gewichtsteile Hochsiedereinsatz, werden 20 bis 30 Gewichtsteile eines Gemisches aus n- und iso-Aldehyd zurückgewonnen.

### Beispiel + Massenbilanz

Ein rohes Hydroformylierungsprodukt aus der Umsetzung von Propylen mit Synthesegas unter Verwendung einer wässrigen Lösung enthaltend Rhodium und Triphenylphosphintrisulfonat wurde gemäß der Arbeitsvorschrift nach US 4,684,750 A1 erhalten und in der ersten Destillationskolonne in iso-Butyraldehyd und n-Butyraldehyd aufgetrennt. Pro Stunde wurde dem Sumpf in der ersten Destillationskolonne 40 Tonnen n-Butyraldehyd mit einem Hochsiedergehalt von etwa 1 Gew.-% entnommen und auf den 10. bis 15. Boden, gerechnet vom Kolonnensumpf, einer zweiten Destillationskolonne gegeben. Die zweite Destillationskolonne wurde bei einer Kopftemperatur von 75 bis 85°C und bei einer Sumpftemperatur von 85 bis 95°C und bei Normaldruck betrieben. Es wurde ein Rücklaufverhältnis von 1 zu 5 eingestellt. Pro Stunde wurden über Kopf 33,3 Tonnen an gereinigtem n-Butyraldehyd entnommen und über den Sumpf 6,7 Tonnen eines Gemisches aus n-Butyraldehyd und Hochsiedern abgeführt und auf den Mittelteil einer dritten Destillationskolonne gegeben. Ebenfalls wurden gleichzeitig die aus zwei entsprechend parallel betriebenen Hydroformylierungsreaktoren erhaltenen Gemische aus n-Butyraldehyd und Hochsiedern auf den Mittelteil der dritten Destillationskolonne gegeben und die dritte Destillationskolonne wurde pro Stunde mit 15 Tonnen Produkt belastet. Bei einer Kopftemperatur von 80°C bis 85°C wurden pro Stunde 14 Tonnen gereinigter n-Butyraldehyd abgezogen, während am Sumpf der dritten Destillationskolonne bei einer Temperatur von 110 bis 115°C pro Stunde 1 Tonne Hochsieder abgeführt wurde. Die dritte Destillationskolonne wurde bei Normaldruck betrieben und wies 30 bis 45 theoretische Böden auf. Die abgeführten Hochsieder, etwa 1 Tonne pro Stunde, wurden auf den Mittelteil einer vierten Destillationskolonne gegeben, in der bei einer Sumpftemperatur von 140 bis 150°C die Hochsieder thermisch gespalten wurden und die flüchtigen Anteile, im Wesentlichen n- und iso-Butyraldehyd mit geringen Mengen an n- und iso-Butanol, bei einer Kopftemperatur von 80 bis 90°C abgeführt wurden. Pro Stunde wurden 200 bis 300 kg flüchtige Produkte abgezogen, während 800 bis 700 kg Hochsieder aus dem Prozess ausgeschleust wurden. Die vierte Destillationskolonne wurde bei Normaldruck betrieben und wies 30 bis 40 theoretische Böden auf.

Durch die erfindungsgemäße thermische Behandlung der Hochsieder in separaten Schritten, wobei zunächst unter Vermeidung der Hochsiederspaltung die in der Hydroformylierungsreaktion gebildeten n- und iso-Aldehyde getrennt werden und anschließend die Hochsieder durch thermische Belastung in ein Gemisch aus n- und iso-Aldehyd gespalten werden, kann die Aldehydselektivität in dem zweiphasigen Hydroformylierungsprozess deutlich gesteigert werden. Aus 100 Gewichtsteilen Hochsiederanfall lassen sich nach dem erfindungsgemäßen Verfahren 20 bis 30 Gewichtsteile eines Gemisches aus n- und iso-Aldehyd zurückgewinnen.

## Patentansprüche

1. Verfahren zur Gewinnung von aliphatischen C₃- bis C₁₀-Aldehyden aus Hochsiedern, die bei der Hydroformylierung der entsprechenden C₂- bis C₉-Olefine in einem heterogenen Reaktionssystem unter Verwendung einer wässrigen Lösung anfallen, die Übergangsmetallkomplexverbindungen der Gruppe VIII des Periodensystems der Elemente mit wasserlöslichen, organischen Phosphor(III)verbindungen gelöst enthält, und wobei man das dem Hydroformylierungsreaktor entnommene Reaktionsgemisch in eine wässrige, Übergangsmetallkomplexverbindung enthaltende Phase und in eine organische Phase trennt, die abgetrennte organische Phase nach Entfernung flüchtiger Bestandteile in einer ersten Destillationskolonne destilliert und ein Kopfprodukt und ein Sumpfprodukt gewinnt, **dadurch gekennzeichnet, dass** man das so gewonnene Sumpfprodukt in eine zweite Destillationskolonne gibt und als Kopfprodukt den n-Aldehyd gewinnt und als Sumpfprodukt ein Gemisch aus dem n-Aldehyd und den Hochsiedern abführt und dass man dieses Sumpfprodukt auf eine dritte Destillationskolonne gibt, die unter solchen Bedingungen betrieben wird, dass noch keine Spaltung der Hochsieder in n-und iso-Aldehyd einsetzt, und dass man das Sumpfprodukt der dritten Destillationskolonne auf eine vierte Destillationskolonne gibt, in der das zugeführte Sumpfprodukt aus der dritten Destillationskolonne bei erhöhter Temperatur gespalten wird, und ein Gemisch aus n- und iso-Aldehyd als Kopfprodukt abzieht.

2. Verfahren gemäß Anspruch 1, **dadurch gekennzeichnet, dass** auf die dritte Destillationskolonne gleichzeitig entsprechende Gemische aus n-Aldehyd und Hochsiedern eingespeist werden, die aus parallel betriebenen und nach dem heterogenen Hydroformylierungsverfahren arbeitenden Reaktoren abgeführt werden.

3. Verfahren gemäß Anspruch 1 oder 2, **dadurch gekennzeichnet, dass** man als Übergangsmetallkomplexverbindungen der Gruppe VIII des Periodensystems der Elemente Rhodiumverbindungen verwendet.

4. Verfahren gemäß einem oder mehreren der Ansprüche 1 bis 3, **dadurch gekennzeichnet, dass** man als wasserlösliche, organische Phosphor(III)verbindungen sulfonierte Triarylphosphine der allgemeinen Formel I 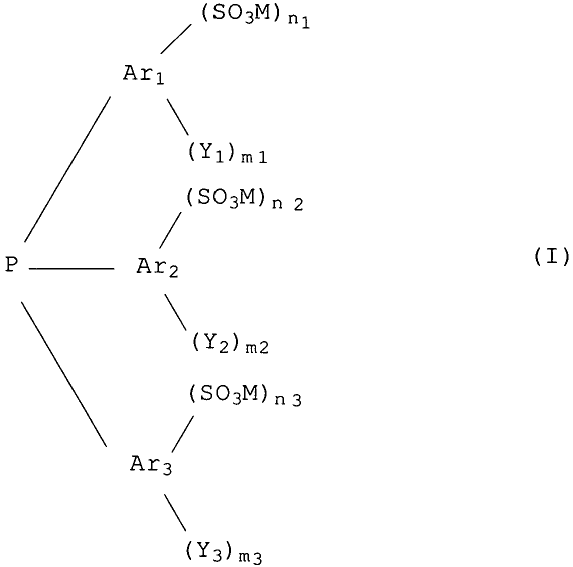 verwendet, in der Ar¹, Ar² und Ar³ gleiche oder verschiedene Arylgruppen mit 6 bis 14 Kohlenstoffatomen bedeuten, die Substituenten Y₁, Y₂ und Y₃ gleiche oder verschiedene geradkettige oder verzweigte Alkyl- oder Alkoxyreste mit 1 bis 4 Kohlenstoffatomen, Chlor, Brom, die Hydroxyl-, Cyanid- oder Nitrogruppe bedeuten, ferner für die Aminogruppe der Formel NR¹R² stehen, in der die Substituenten R¹ und R² gleich oder verschieden sind und Wasserstoff, geradkettige oder verzweigte Alkylgruppen mit 1 bis 4 Kohlenstoffatomen bedeuten, in der M für Lithium, Natrium, Kalium, Magnesium, Calcium oder Barium steht, in der m₁, m₂ und m₃ gleich oder verschieden sind und ganze Zahlen von 0 bis 5 bedeuten, in der n₁, n₂ und n₃ gleich oder verschieden sind und für ganze Zahlen von 0 bis 3 stehen, wobei mindestens eine der Zahlen n₁, n₂ und n₃ gleich oder größer 1 ist.

5. Verfahren gemäß einem oder mehreren der Ansprüche 1 bis 4, **dadurch gekennzeichnet, dass** als C₂-C₉-Olefin Ethylen, Propylen, Buten-1 oder Gemische enthaltend Buten-1 und Buten-2 verwendet werden.

6. Verfahren gemäß einem oder mehreren der Ansprüche 1 bis 5 **dadurch gekennzeichnet, dass** im Falle von Propylen als C₂- bis C₉-Olefin die dritte Destillationskolonne bei einer Kopftemperatur von 75 bis 95°C, bei einer Sumpftemperatur von 105 bis 120°C und bei Normaldruck betrieben wird.

7. Verfahren gemäß Anspruch 6, **dadurch gekennzeichnet, dass** die dritte Destillationskolonne bei einer Kopftemperatur von 80 bis 90°C, und bei einer Sumpftemperatur von 110 bis 115°C betrieben wird.

8. Verfahren gemäß einem oder mehreren der Ansprüche 1 bis 7, **dadurch gekennzeichnet, dass** im Falle von Propylen als C₂- bis C₉-Olefin die vierte Destillationskolonne bei einer Kopftemperatur von 70 bis 100°C, bei einer Sumpftemperatur von 130 bis 160°C und bei Normaldruck betrieben wird.

9. Verfahren gemäß Anspruch 8, **dadurch gekennzeichnet, dass** die vierte Destillationskolonne bei einer Kopftemperatur von 80 bis 90°C und bei einer Sumpftemperatur von 140 bis 150°C betrieben wird.

10. Verfahren gemäß einem oder mehreren der Ansprüche 1 bis 9, **dadurch gekennzeichnet, dass** anstelle der ersten Destillationskolonne eine Mehrzahl von hintereinandergeschalteten Destillationskolonnen zur Abtrennung des iso-Aldehyds verwendet werden.

## Claims

1. Process for obtaining aliphatic C₃-C₁₀-aldehydes from high boilers formed in the hydroformylation of the corresponding C₂-C₉-olefins in a heterogeneous reaction system using an aqueous solution containing dissolved transition metal complexes of group VIII of the Periodic Table of the Elements with water-soluble, organic phosphorus(III) compounds, where the reaction mixture taken from the hydroformylation reactor is separated into an aqueous phase containing transition metal complex and an organic phase, the organic phase which has been separated off is, after removal of volatile constituents, distilled in a first distillation column and an overhead product and a bottom product are obtained, **characterized in that** the bottom product obtained in this way is introduced into a second distillation column and the n-aldehyde is obtained as overhead product and a mixture of the n-aldehyde and the high boilers is discharged as bottom product and **in that** this bottom product is introduced into a third distillation column which is operated under such conditions that no dissociation of the high boilers into n-aldehyde and iso-aldehyde occurs and **in that** the bottom product of the third distillation column is introduced into a fourth distillation column in which the bottom product from the third distillation column which has been fed in is dissociated at elevated temperature and a mixture of n-aldehyde and iso-aldehyde is taken off as overhead product.

2. Process according to Claim 1, **characterized in that** corresponding mixtures of n-aldehyde and high boilers which are discharged from reactors operated in parallel and according to the heterogeneous hydroformylation process are at the same time fed into the third distillation column.

3. Process according to Claim 1 or 2, **characterized in that** rhodium compounds are used as transition metal complexes of group VIII of the Periodic Table of the Elements.

4. Process according to one or more of Claims 1 to 3, **characterized in that** sulfonated triarylphosphines of the formula I 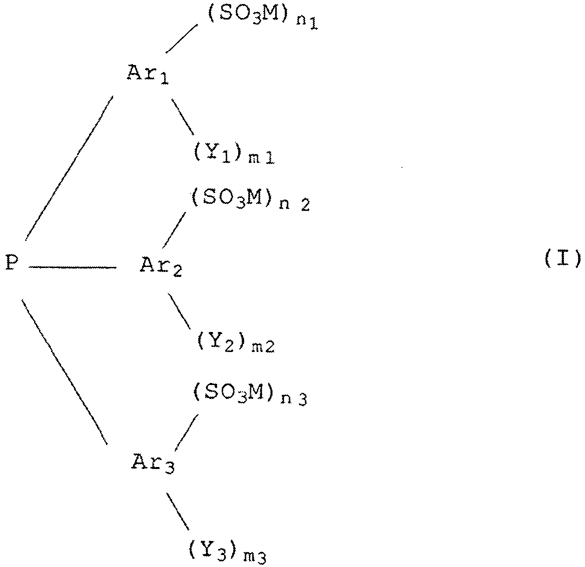 where Ar¹, Ar² and Ar³ are identical or different aryl groups having from 6 to 14 carbon atoms, the substituents Y₁, Y₂ and Y₃ are identical or different straight-chain or branched alkyl or alkoxy radicals having from 1 to 4 carbon atoms, chlorine, bromine, the hydroxyl, cyanide or nitro group, also the amino group of the formula NR¹R² in which the substituents R¹ and R² are identical or different and are hydrogen or straight-chain or branched alkyl groups having from 1 to 4 carbon atoms, M is lithium, sodium, potassium, magnesium, calcium or barium, m₁, m₂ and m₃ are identical or different and are integers from 0 to 5, n₁, n₂ and n₃ are identical or different and are integers from 0 to 3, with at least one of the numbers n₁, n₂ and n₃ being equal to or greater than 1,
are used as water-soluble, organic phosphorus(III) compounds.

5. Process according to one or more of Claims 1 to 4, **characterized in that** ethylene, propylene, 1-butene or a mixture containing 1-butene and 2-butene are used as C₂-C₉-olefin.

6. Process according to one or more of Claims 1 to 5, **characterized in that** in the case of propylene as C₂-C₉-olefin, the third distillation column is operated at a temperature at the top of from 75 to 95°C, at a temperature at the bottom of from 105 to 120°C and at atmospheric pressure.

7. Process according to Claim 6, **characterized in that** the third distillation column is operated at a temperature at the top of from 80 to 90°C and at a temperature at the bottom of from 110 to 115°C.

8. Process according to one or more of Claims 1 to 7, **characterized in that** in the case of propylene as C₂-C₉-olefin, the fourth distillation column is operated at a temperature at the top of from 70 to 100°C, at a temperature at the bottom of from 130 to 160°C and at atmospheric pressure.

9. Process according to Claim 8, **characterized in that** the fourth distillation column is operated at a temperature at the top of from 80 to 90°C and at a temperature at the bottom of from 140 to 150°C.

10. Process according to one or more of Claims 1 to 9, **characterized in that** a plurality of distillation columns connected in series are used instead of the first distillation column for separating off the iso-aldehyde.

## Revendications

1. Procédé pour la production d'aldéhydes aliphatiques en C₃-C₁₀ à partir de fractions lourdes qui sont obtenues lors de l'hydroformylation des C₂-C₉-oléfines correspondantes dans un système de réaction hétérogène avec utilisation d'une solution aqueuse, qui contient des composés complexes de métaux de transition du groupe VIII du système périodique des éléments avec des composés solubles dans l'eau, organiques, du phosphore (III), sous forme dissoute et le mélange réactionnel prélevé du réacteur hydroformylation étant séparé en une phase aqueuse, contenant le composé complexe de métaux de transition, et en une phase organique, la phase organique séparée étant distillée, après élimination des constituants volatils, dans une première colonne de distillation et un produit de tête et un produit de fond étant obtenus, **caractérisé en ce qu'**on alimente le produit de fond ainsi obtenu sur une deuxième colonne de distillation et on obtient, comme produit de tête, le n-aldéhyde et, comme produit de fond, un mélange constitué par le n-aldéhyde et la fraction lourde, et **en ce qu'**on alimente ce produit de fond sur une troisième colonne de distillation, qui est exploitée dans des conditions telles qu'une dissociation de la fraction lourde en n-aldéhyde et en iso-aldéhyde ne démarre pas encore, et **en ce qu'**on alimente le produit de fond de la troisième colonne de distillation sur une quatrième colonne de distillation, dans laquelle le produit de fond alimenté provenant de la troisième colonne de distillation est dissocié à température augmentée et on soutire un mélange de n-aldéhyde et d'iso-aldéhyde comme produit de tête.

2. Procédé selon la revendication 1, **caractérisé en ce qu'**on injecte, sur la troisième colonne de distillation, simultanément des mélanges correspondants de n-aldéhyde et de fraction lourde, qui sont évacués de réacteurs fonctionnant en parallèle et travaillant selon le procédé d'hydroformylation hétérogène.

3. Procédé selon la revendication 1 ou 2, **caractérisé en ce qu'**on utilise des composés de rhodium comme composés complexes de métaux de transition du groupe VIII du système périodique des éléments.

4. Procédé selon l'une ou plusieurs des revendications 1 à 3, **caractérisé en ce qu'**on utilise des triarylphosphines sulfonées de formule générale I comme composés solubles dans l'eau, organiques, du phosphore (III) 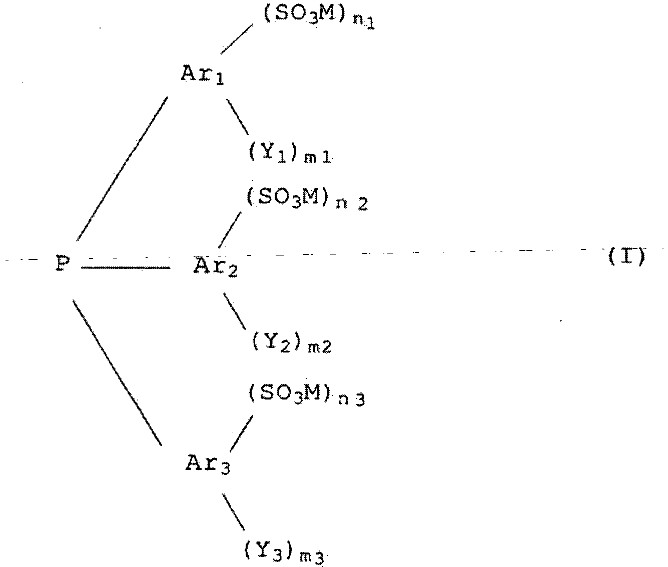 dans laquelle Ar¹, Ar² et Ar³ signifient des groupes aryle identiques ou différents comprenant 6 à 14 atomes de carbone, les substituants Y¹, Y² et Y³ signifient des radicaux alkyle ou alcoxy identiques ou différents, linéaires ou ramifiés comprenant 1 à 4 atomes de carbone, chlore, brome, le groupe hydroxyle, cyanure ou nitro, représentent en outre le groupe amino de formule NR¹R², dans laquelle les substituants R¹ et R² sont identiques ou différents et signifient hydrogène, des groupes alkyle linéaires ou ramifiés comprenant 1 à 4 atomes de carbone, M représente lithium, sodium, potassium, magnésium, calcium ou baryum, m₁, m₂ et m₃ sont identiques ou différents et signifient des nombres entiers de 0 à 5, n₁, n₂ et n₃ sont identiques ou différents et représentent des nombres entiers de 0 à 3, au moins un des nombres n₁, n₂ et n₃ étant égal ou supérieur à 1.

5. Procédé selon l'une ou plusieurs des revendications 1 à 4, **caractérisé en ce qu'**on utilise, comme C₂-C₉-oléfine, l'éthylène, le propylène, le butène-1 ou des mélanges contenant du butène-1 et du butène-2.

6. Procédé selon l'une ou plusieurs des revendications 1 à 5, **caractérisé en ce que** dans le cas de propylène comme C₂-C₉-oléfine, la troisième colonne de distillation est exploitée à une température de tête de 75 à 95°C, à une température de fond de 105 à 120°C et à pression normale.

7. Procédé selon la revendication 6, **caractérisé en ce que** la troisième colonne de distillation est exploitée à une température de tête de 80 à 90°C et à une température de fond de 110 à 115°C.

8. Procédé selon l'une ou plusieurs des revendications 1 à 7, **caractérisé en ce que** dans le cas de propylène comme C₂-C₉-oléfine, la quatrième colonne de distillation est exploitée à une température de tête de 70 à 100°C, à une température de fond de 130 à 160°C et à pression normale.

9. Procédé selon la revendication 8, **caractérisé en ce que** la quatrième colonne de distillation est exploitée à une température de tête de 80 à 90°C et à une température de fond de 140 à 150°C.

10. Procédé selon l'une ou plusieurs des revendications 1 à 9, **caractérisé en ce qu'**on utilise, au lieu de la première colonne de distillation, une multitude de colonnes de distillation disposées les unes derrière les autres pour la séparation de l'iso-aldéhyde.
